Europäisches Patentamt

European Patent Office

Office européen des brevets

(19)

(11) Veröffentlichungsnummer: **0 019 043**
**B1**

(12) **EUROPÄISCHE PATENTSCHRIFT**

(45) Veröffentlichungstag der Patentschrift:
25.05.83

(51) Int. Cl.³: **F 16 B 33/04, A 61 F 1/00**

(21) Anmeldenummer: **80101208.9**

(22) Anmeldetag: **10.03.80**

(54) Kohlenstoffschraube, Verfahren zu deren Herstellung und deren Verwendung.

(30) Priorität: **19.05.79 DE 2920357**

(43) Veröffentlichungstag der Anmeldung:
**26.11.80 Patentblatt 80/24**

(45) Bekanntmachung des Hinweises auf die Patenterteilung:
**25.05.83 Patentblatt 83/21**

(84) Benannte Vertragsstaaten:
**AT CH FR GB**

(56) Entgegenhaltungen:
**AT-B-349 620**
**DE-A-2 705 336**
**GB-A-1 527 067**

(73) Patentinhaber: **SIGRI ELEKTROGRAPHIT GMBH,**
**Werner von Siemens-Strasse 18, D-8901 Meitingen (DE)**

(72) Erfinder: **Gruber, Udo, Chem. Ing. grad., Eschenweg 4,**
**D-8851 Thierhaupten (DE)**
Erfinder: **Loos, Wolfgang, Ing. grad., Studentenweg 6,**
**D-7932 Munderkingen (DE)**
Erfinder: **Gerstenberger, Friedrich, Prof. Dipl.-Ing.,**
**Grabenstrasse 11, D-7321 Dürnau (DE)**

Kohlenstoffschraube, Verfahren zu deren Herstellung und deren Verwendung

Die Erfindung betrifft eine Kohlenstoffasern enthaltende Kohlenstoffschraube, in welcher die Kohlenstoffasern im Schraubenkern und im Schraubengewinde verschieden orientiert sind, ein Verfahren zur Herstellung der Schraube und die Verwendung der Schraube.

Zur kraftschlüssigen Verbindung von Elementen aus Kohlenstoff oder Graphit und zum Verbinden derartiger Elemente mit Konstruktionsteilen aus einem anderen Werkstoff sind Schrauben bekanntgeworden, die aus Kohlenstoff bestehen, der durch Kohlenstoffasern verstärkt ist. Zur Herstellung der Schrauben hat man zunächst Platten oder Zylinder aus einem die Kohlenstoffaser in einer im wesentlichen unidirektionalen Orientierung enthaltenden Verbundwerkstoff angefertigt und die Schrauben dann aus diesen Rohlingen herausgearbeitet, wobei die Schraubenlängsachse mit der Richtung der Kohlenstoffasern zusammenfällt. Nachteilig ist die verhältnismäßig geringe Scherfestigkeit derartiger Schrauben, so daß unter Last einzelne Gewindegänge leicht abscheren. Durch die DE-A-2 705 336 ist eine Kohlenstoffschraube bekannt, die eine verbesserte Scherfestigkeit aufweist. In dieser Schraube sind die Verstärkungsfasern im Schraubenkern parallel zur Längsachse orientiert, im Gewinde verlaufen sie unter einem spitzen Winkel zu der Längsachse. In einer derartig ausgebildeten Schraube werden Zug- und Biegespannungen vorzugsweise vom Schraubenkern und Scherspannungen vorzugsweise von den Gewindegängen aufgenommen und man erreicht Scherfestigkeiten, die fast doppelt so groß sind wie die Scherfestigkeit ausschließlich unidirektional angeordnete Verstärkungsfasern enthaltender Schrauben. Trotz der verbesserten Scherfestigkeit konnten die Schrauben nicht unter allen Belastungsbedingungen befriedigen, vor allem weil die Haftung zwischen den auf den Kern gewickelten Gewindegängen und dem Schraubenkern nicht immer ausreichte. Nach der AT-B-349 620 ist schließlich ein mit einem Schraubgewinde versehener Prothesenstiel aus mit Kohlenstoffasern verstärktem Kohlenstoff bekannt. Die Kohlenstoffasern sind bevorzugt in mehreren Scharen mit untereinander parallelen Fasern angeordnet und die Scharen gegeneinander geneigt. Die Schraube besteht entsprechend aus mehreren Faserschichten mit verschiedener Faserorientierung, wobei die Längserstreckung der Fasern zweckmäßig mit den Hauptbelastungsrichtungen zusammenfällt.

Der Erfindung liegt daher die Aufgabe zugrunde, die Festigkeit und die Betriebssicherheit von Kohlenstoffasern enthaltenden Kohlenstoffschrauben zu verbessern.

Die Aufgabe wird mit einer Kohlenstoffschraube der eingangs genannten Art dadurch gelöst, daß der zylindrische Schraubenkern Faserschichten als Verstärkungselemente enthält und eine die Mantelfläche durchschneidende schraubenlinienförmig verlaufende, die Gewindewurzel bildende Ausnehmung aufweist und die Fasern in der Gewindewurzel und im Gewinde parallel zu der Schraubenlinie verlaufen.

Der Schraubenkern enthält aus Kohlenstoffasern gebildete Faserschichten, wobei die Fasern innerhalb einer Schicht parallel zueinander angeordnet sind und schief zur Längsachse des Schraubenkerns verlaufen. Insbesondere ist auch der Neigungswinkel der Fasern verschiedener Faserschichten verschieden. Beispielsweise weisen die Fasern einer ersten Faserschicht einen Neigungswinkel von 30° auf und in einer zweiten Schicht beträgt der Neigungswinkel beispielsweise 150°. Die Fasergebilde können auch aus Kohlenstoffgewebe gebildet sein, z. B. in Leinwandbindung, wobei beispielsweise die Fasern der ersten Schicht um 30 und 120° und die der zweiten Schicht um 60 und 150° gegen die Schraubenachse geneigt sind usw. Die Festigkeit bei einer einachsigen Belastung parallel zur Schraubenachse ist bei dieser Anordnung der Verstärkungselemente zwar geringer als von unidirektional verstärkten Schrauben, da aber in der Regel eine mehrachsige Beanspruchung vorherrscht, ist im ganzen ein breiteres Anwendungsspektrum zugänglich. Vorteilhaft ist auch die einfachere Bearbeitung des Schraubenkerns, etwa das Ausschneiden der Gewindewurzel. Durch Verankerung der Verstärkungsfasern des Gewindes in dieser Wurzel wird eine wesentliche Verbesserung der Scherfestigkeit und entsprechend der zulässige Belastungsbereich der Schraube vergrößert.

Zur Herstellung der Kohlenstoffschraube werden mit einem Kunstharz, wie z. B. Furan- oder Phenolharz beschichtete Kohlenstoff- oder Graphitfäden in paralleler Orientierung zu Schichten ausgelegt und das Binderharz partiell gehärtet. Die Faserschichten werden übereinandergestapelt und die einzelnen Schichten dabei so gegeneinander gedreht, daß die Fäden in den einzelnen Gewebeschichten in verschiedene Richtungen verlaufen. Der Schichtstapel wird dann bevorzugt unter einem erhöhten Druck zur vollständigen Härtung des Kunstharzes auf eine Temperatur zwischen etwa 120 und 200° C erhitzt, woran sich zur Carbonisierung des Harzes eine zweite Wärmebehandlung in einer inerten Atmosphäre bei einer Temperatur bis zu etwa 1000° C anschließt. Aus dem nun im wesentlichen aus Kohlenstoff bestehenden Körper wird ein zylindrischer Schraubenkern herausgearbeitet und in die Mantelfläche des Kerns eine schraubenlinienförmig verlaufende Ausnehmung gefräst oder gedreht. Diese Schraubenlinie bildet die Wurzel des Gewindes in die im Wickelverfahren aus kunstharzimprägnierten Kohlenstoff- oder Graphitgarnen oder Rovings eine aus mehreren Lagen bestehende Schicht bis zum gewünschten Außendurchmesser der Schraube aufgezogen wird. Die Kohlenstoffasern verlaufen in dieser Schicht parallel zu der schraubenlinienförmigen Ausnehmung und tangential zur Mantelfläche. Zum Härten und Carbonisieren des Kunstharzes wird der Rohling zunächst auf eine Temperatur von 120 bis 200°C und daran anschließend unter inerten Bedingungen auf etwa 1000°C erhitzt. Nach dem Carbonisieren des Harzes wird in bekannter Weise in die Wickelschicht parallel zur Schraubenlinie ein Gewinde geschnitten. Alternativ kann die Wickelschicht auch auf den gehärteten aber noch nicht carbonisierten Kern aufgebracht werden und es ist ebenfalls möglich, die Schraubenrohlinge oder auch die Schraubenkerne allein in bekannter Weise mit einem carbonisierbaren Imprägniermittel wie Steinkohlenteerpech oder Kunstharze ein oder mehrmals zu imprägnieren, wobei die Körper nach jeder Imprägnierung zum Carbonisieren des Imprägniermittels auf etwa 1000°C erhitzt werden müssen.

In der Tabelle sind die Ergebnisse von Ausreißversuchen mit erfindungsgemäßen Schrauben aus Stahlmuttern wiedergegeben. Der Gewindedurchmesser betrug 12,7 mm.

| Nr. | Bruchkraft (kN) |
| --- | --- |
| 1 | 9,4 |
| 2 | 11,4 |
| 3 | 13,3 |
| 4 | 11,4 |
| 5 | 10,5 |
| 6 | 10,0 |
| 7 | 9,7 |
| 8 | 12,2 |
| 9 | 10,2 |
| 10 | 11,1 |

Die Bruchkraft von Schrauben aus mit Kohlenstoffasern verstärktem Kohlenstoff nach DE-OS 2 705 336 betrug 2 bis 3 kN.

**Patentansprüche**

1. Kohlenstoffasern enthaltende Kohlenstoffschraube, in welcher die Kohlenstoffasern im Schraubenkern und im Schraubengewinde verschieden orientiert sind, dadurch gekennzeichnet, daß der zylindrische Schraubenkern Faserschichten als Verstärkungselemente enthält und eine die Mantelfläche durchschneidende schraubenlinienförmig verlaufende, die Gewindewurzel bildende Ausnehmung aufweist und die Fasern in der Gewindewurzel und im Gewinde parallel zu der Schraubenlinie verlaufen.

2. Verfahren zum Herstellen einer Kohlenstoffschraube nach Patentanspruch 1, bei welchem mehrere mit einem härtbaren Kunstharz imprägnierte gegeneinander verdrehte Schichten aus Kohlenstoffasern übereinandergestapelt und unter einem erhöhten Druck zum Härten und Carbonisieren des Harzes einer thermischen Behandlung unterzogen werden, dadurch gekennzeichnet, daß

a) aus dem Schichtenstapel ein zylindrischer Schraubenkern herausgearbeitet und die Mantelfläche mit einer schraubenlinienförmig verlaufenden Ausnehmung versehen wird,

b) mit einem härtbaren Kunstharz imprägnierte Kohlenstoffäden, ausgehend von der Ausnehmung parallel zu der Schraubenlinie auf den Kern gewickelt werden, und

c) der Rohling zum Härten und Carbonisieren des Kunstharzes einer zweiten Wärmebehandlung unterzogen und anschließend in die Wickelschicht parallel zur Schraubenlinie ein Gewinde geschnitten wird.

3. Verwendung einer Kohlenstoffschraube nach den Patentansprüchen 1 und 2 als schraubbaren Prothesenschaft für eine Hüftgelenkendoprothese.

**Claims**

1. Carbon fibre reinforced carbon screw comprising a cylindrical core being reinforced by layers of carbon fibres, the generated surface thereof being dissected by a helical wound groove forming the base of the screwthread and reinforcing carbon fibres within the groove and the thread extending parallel thereto.

2. Method for producing a carbon fibre reinforced carbon screw as claimed in claim 1, comprising

a) forming a pile containing a plurality of prepreg sheets consisting of a curable resin and carbon fibres, the alignment of the carbon fibres in every sheet being different from each adjacent sheet, and curing and carbonising the resin by heating the pile,

b) machining from the pile a cylindrical core and cutting a helical wound groove in the generated surface thereof,

c) winding resin impregnated carbon fibre strands parallel to the hilical extending groove around the core,

d) curing and carbonising the resin and cutting threads parallel to the helical extending groove in the wound fibre layer.

3. Use of the carbon fibre reinforced carbon screw as claimed in claims 1 and 2 as screwable prothesis shaft of a hip joint endoprothesis.

## Revendications

1. Vis en carbone contenant des fibres de carbone, vis dans laquelle les fibres de carbone sont orientées de façon différente dans le coeur de la vis, et dans le filetage de la vis, vis caractérisée en ce que le coeur cylindrique de la vis contient des couches de fibres comme éléments de renforcement, et comporte un évidement, recoupant la surface enveloppe, s'étendant en forme d'hélice et constituant la base du filetage, cependant que les fibres dans cette base du filetage et dans le filetage, sont parallèles à cette hélice.

2. Procédé pour réaliser une vis en carbone selon la revendication 1, procédé dans lequel plusieurs couches de fibres de carbone torsadées les unes par rapport aux autres et imprégnées d'une résine synthétique durcissable, sont empilées les unes sur les autres et sont soumises à un traitement thermique sous une pression élevée pour durcir et carbonier la résine, procédé caractérisé en ce que:

a) à partir de l'empilement de couches est usiné un coeur de vis cylindrique, tandis que la surface enveloppe est munie d'un évidement s'étendant en forme d'hélice,

b) des fils de carbone imprégnés d'une résine synthétique durcissable sont enroulés sur le coeur, parallèlement à l'hélice, en partant de l'évidement,

c) l'ébauche est soumise à un second traitement thermique pour durcir et carboniser la résine synthétique, puis un filetage est usiné dans la couche d'enroulement parallèle à l'hélice.

3. Utilisation d'une vis en carbone selon les revendications 1 et 2, comme fût de prothèse susceptible d'être vissé pour une endoprothèse de l'articulation de la hanche.